# EUROPEAN PATENT SPECIFICATION

(11) **EP 3 975 951 B1**
(45) Date of publication and mention of the grant of the patent: **17.07.2024**
(21) Application number: 20747180.6
(22) Date of filing: 02.06.2020
(51) Int. Cl.: A61F 9/007, A61L 31/04, A61L 31/14

(54) **NON-DEGRADING SWELLABLE POLYMERS AS MATERIALS FOR BIOMEDICAL DEVICES**
NICHTABBAUBARE QUELLBARE POLYMERE ALS MATERIALIEN FÜR BIOMEDIZINISCHE VORRICHTUNGEN
POLYMÈRES GONFLABLES SANS DÉGRADATION EN TANT QUE MATÉRIAUX POUR DISPOSITIFS BIOMÉDICAUX

(30) Priority: 03.06.2019 US 201962856447 P
(43) Date of publication of application: 06.04.2022
(73) Proprietor: Innfocus, Inc., Miami, FL 33186 (US)
(72) Inventor: PINCHUK, Leonard, Miami, FL 33176 (US); KWON, Yongmoon, Weston, FL 33332 (US)
(74) Representative: Jones Day
(86) International application number: PCT/US2020/035688
(87) International publication number: WO 2020/247365

(56) References cited:
- WO-A2-2013/069018
- RU-C1- 2 578 424
- C. T. CHIANG ET AL: "A Poly-HEMA Based Aqueous Humor Draining Device", JOURNAL OF APPLIED BIOMATERIALS, 1 January 1990 (1990-01-01), pages 321 - 327, XP055723000, Retrieved from the Internet <URL:https://onlinelibrary.wiley.com/reader/content/10.1002/jab.770010408/format/pdf/OEBPS/pages/bg1.png> [retrieved on 20200817]
- JING WANG ET AL: "Aqueous shunt implantation in glaucoma", TAIWAN JOURNAL OF OPHTHALMOLOGY, vol. 7, no. 3, 1 January 2017 (2017-01-01), NL, pages 130, XP055723006, ISSN: 2211-5056, DOI: 10.4103/tjo.tjo_35_17

## Description

### CROSS-REFERENCE TO RELATED APPLICATIONS

This application claims the benefit of U.S. Provisional Application No. 62/856,447, filed June 3, 2019, and entitled "Non-Degrading Swellable Polymers as Materials for Biomedical Devices".

### FIELD OF DISCLOSURE

The present disclosure relates to medical devices, such as intraocular shunts, that are made from materials comprising non-degradable, swellable polymer hydrogels, and methods of making the same.

### BACKGROUND

Intraocular shunts are used for glaucoma treatment. The shunts can be placed *ab interno* (introduced from within the anterior chamber) or *ab externo* (introduced through the conjunctiva or sclera) shunting fluid from the anterior chamber to below the conjunctiva or below Tenon's capsule. Regardless of outflow location, both paths result in subconjunctival blebs. The shunt can be inserted into place using a needle-like inserter and is designed to swell in place to seal between the needle track and the shunt and direct flow through the lumen of the device. The lumen is designed as a flow resistor or flow restrictor to both prevent hypotony (deflation of the eye) as well as to prevent hypertrophy (excessive pressure in the eye).

Typical glaucoma shunts that swell into place are simple tubes often made from glutaraldehyde crosslinked porcine gelatin. The problem with a shunt made from collagen is that tissue can grow into the wall of the proteinaceous shunt, macrophages and water oxidize and hydrolyze the proteinaceous shunt and it generates byproducts, provokes inflammation, becomes brittle and fragile, and if manipulated, can break off. Eventually, the shunt becomes totally overgrown and ceases to function, thereby requiring further treatment to lower intraocular pressure (IOP) in order to stop the progression of glaucoma and thwart vision loss.

Accordingly, intraocular shunts and materials to fabricate the same are needed that are not subject to backbone degradation and will survive in the anatomical locations that are desirable for intraocular shunt placement. More broadly, there is a need for materials for additional types of implantable medical devices that are required to expand upon implantation and also maintain biostability for periods of time without significant degradation.

WO2013/069018 A2 discloses an intraocular shunt, which can be made of crosslinked polymer which can be a copolymer that includes methacrylic acid monomers.

### SUMMARY

In a first aspect, disclosed herein is an intraocular shunt formed from a material comprising a non-degrading swellable polymer having the formula: 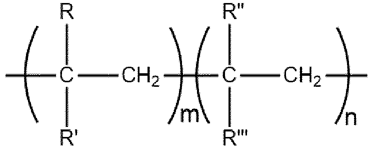 wherein R and R" are methyl; R' is -COOR¹; R¹ is alkyl; and R'" is a carboxylic acid; and the ratio of m:n is from about 99.99:0.01 to about 90:10; wherein the intraocular shunt has a first diameter in a dehydrated state and a second diameter in a hydrated state, wherein the second diameter is greater than the first diameter.

### BRIEF DESCRIPTION OF THE DRAWINGS

The following detailed description of specific embodiments of the disclosure will be better understood when read in conjunction with the appended drawings. For the purpose of illustrating the disclosure, specific embodiments are shown in the drawings. It should be understood, however, that the disclosure is not limited to the precise arrangements and instrumentalities of the embodiments shown in the drawings.
**FIG. 1A** shows an intraocular shunt in a dehydrated state in accordance with an embodiment of the disclosure.
**FIG. 1B** shows an intraocular shunt in a hydrated state in accordance with an embodiment of the disclosure.
**FIG. 2** shows an intraocular shunt inserted into the lumen of a needle in accordance with an embodiment of the disclosure.
**FIG. 3** shows an intraocular shunt placed in tissue in a hydrated state in accordance with an embodiment of the disclosure.
**FIG. 4** shows a graph of water content and swollen volume of gels in accordance with an embodiment of the disclosure.
**FIG. 5** shows a pHEMA tube in a hydrated state (top) and dehydrated state (bottom) in accordance with an embodiment of the disclosure.

### DETAILED DESCRIPTION OF THE INVENTION

The present disclosure relates to a family of hydrogels for use as materials in intraocular shunts, e.g., glaucoma shunts, as defned by the claims. The hydrogels are not subject to backbone degradation and will survive in the anatomical locations that are desirable for a glaucoma shunt. The hydrogels can be used to fabricate devices, such as implantable devices. The hydrogels of the present disclosure and devices fabricated therefrom swell when exposed to certain fluids, i.e., biological fluids; however in contrast to known devices, the hydrogels and devices of the present disclosure will not degrade or embrittle when exposed to fluids and therefore are suitable as implantable devices in subjects. The hydrogel comprises a completely aliphatic polymer, i.e., a polymer that has no double bonds in the polymer backbone. The aliphatic polymer is not subject to degradation upon exposure to a fluid, i.e., a biological fluid. The aliphatic polymer will not undergo an S_{N}1 hydrolysis reaction and therefore will undergo little, or no, degradation when implanted into a subject. Accordingly, the hydrogels swell upon hydration but do not degrade when inserted into the eye of a patient. The hydrogel structure comprises features that promote swelling upon hydration and features that restrict degradation. One non-limiting advantage of the present disclosure is the ability to manufacture a device, e.g., an intraocular shunt, that swells into place upon insertion into the eye, thus eliminating the need for fins or barbs to keep the device from migrating into or out of the eye. Moreover, devices made from the materials disclosed herein can be placed in the eye both in an ab-interno or an in ab-externo manner. Importantly, simple ab-externo implantation can be performed in a physician's office, rather than in the operating room, which decreases the burden on both patients and physicians. Another non-limiting advantage of the hydrogels of the present disclosure (and devices fabricated therefrom) is the ability to fabricate devices to suitable specifications to fit through a certain gauge needle lumen, e.g., a 27 gauge needle lumen. The hydrogels of the present disclosure can be fabricated to the exact dimensions (i.e, inner and outer diameters and length) required to fit through the desired gauge needle lumen.

### Polymer Backbone

In certain embodiments, the material is a hydrogel that comprises a polymer which comprises a backbone in which the majority of the polymer linkages along the polymer chain are alternating quaternary and secondary carbon atoms. The term "secondary" carbons, as used herein, means carbon atoms which are bonded to two other carbon atoms and two other hydrogens. The term "quaternary" carbons, as used herein, means carbon atoms which are bonded to four other carbon atoms and no hydrogens. In certain embodiments, this alternating quaternary and secondary carbon atom configuration promotes polymer stability. In certain embodiments, the polymer backbones comprising alternating quaternary and secondary carbon atoms are resistant to oxidation, hydrolysis, or embrittlement in biological environments. In certain embodiments, the polymer backbones comprising alternating quaternary and secondary carbon atoms will not oxidize, hydrolyze, or embrittle in biological environments. In certain embodiments, the polymers are configured to minimize or prevent cracking, crazing or degradation thereof when they are implanted *in vivo* or otherwise subjected to degradation conditions.

In certain embodiments, the polymer comprises a backbone in which the majority of the polymer linkages along the polymer chain are alternating quaternary and secondary carbon atoms. In certain embodiments, the polymer comprises a backbone in which from about 25% to about 100%, from about 50% to about 100%, from about 75% to about 100%, from about 90% to about 100%, or from about 95% to about 100% of the polymer linkages along the polymer chain are alternating quaternary and secondary carbon atoms. In certain embodiments, the polymer comprises a backbone in which from about 98% to about 100% of the polymer linkages along the polymer chain are alternating quaternary and secondary carbon atoms. In certain embodiments, the polymer comprises a backbone in which about 50%, 55%, 60%, 65%, 70%, 75%, 80%, 85%, 90%, 95%, 96%, 97%, 98%, 99%, or 100% of polymer linkages along the polymer chain are alternating quaternary and secondary carbon atoms. In certain embodiments, the polymer comprises a backbone in which about 95% of polymer linkages along the polymer chain are alternating quaternary and secondary carbon atoms. In certain embodiments, the polymer comprises a backbone in which about 97% of polymer linkages along the polymer chain are alternating quaternary and secondary carbon atoms. In certain embodiments, the polymer comprises a backbone in which about 99% of polymer linkages along the polymer chain are alternating quaternary and secondary carbon atoms. In certain embodiments, the polymer comprises a backbone in which greater than about 50%, 55%, 60%, 65%, 70%, 75%, 80%, 85%, 90%, 95%, 96%, 97%, 98%, or 99% of polymer linkages along the polymer chain are alternating quaternary and secondary carbon atoms.

In certain embodiments, the material is a hydrogel that comprises a polymer which comprises a backbone in which the majority of the polymer linkages along the polymer chain are alternating tertiary and secondary carbon atoms. The term "tertiary" carbons, as used herein, means carbon atoms which are bonded to three other carbon atoms and one hydrogen atom. One of ordinary skill in the art would know that these polymers can support conjugated double bonds on their backbones which can lead to bio-instability; however, in certain embodiments, the polymers are biostable. Accordingly, in certain embodiments, the polymer in which the majority of the polymer linkages along the polymer chain are alternating tertiary and secondary carbon atoms is biostable.

In certain embodiments, the hydrogels comprise non-degrading swellable polymers comprising a mixture of monomers that include swellable monomers and hydrophobic monomers. In certain embodiments, the hydrogels are formed from a mixture of monomers that comprises both hydrophobic and swellable monomers.

### Hydrophobic monomers

As used herein, the term "hydrophobic monomer" means that the monomer, upon polymerization thereof, substantially prevents water from entering the resulting polymeric structure. In certain embodiments, the hydrophobic monomer is a non-swellable monomer. In certain embodiments, upon polymerization of the hydrophobic monomer, the resulting polymer is a non-swellable polymer. In certain embodiments, the hydrophobic monomer produces a polymer that substantially prevents water from entering the resulting polymeric structure such that the polymer does not swell upon hydration. In certain embodiments, the hydrophobic monomer includes side groups comprising hydrophobic groups. In certain embodiments, the hydrophobic monomer includes side groups that do not comprise hydrophilic groups. One of ordinary skill in the art would understand that the quaternary carbons on the polymer backbone will include two side groups. In certain embodiments, both of the side groups on the quaternary carbons of the hydrophobic monomer comprises hydrophobic groups. In certain embodiments, the hydrophobic group is selected from the group consisting of alkyl, cycloalkyl, aryl, halo, alkylaryl, and alkoxy. In certain embodiments, the hydrophobic group is selected from C₁₋₁₂ alkyl, C₁₋₁₀ alkyl, C₁₋₈ alkyl, C₁₋₆ alkyl, C₁₋₄ alkyl, C₂₋₄ alkyl, C₂₋₆ alkyl, C₂₋₈ alkyl, C₂₋₁₀ alkyl, C₂₋₁₂ alkyl, methyl, ethyl, propyl, butyl, and hexyl, each of which can be linear, branched or cyclic, each of which can be further substituted with an aryl group. In certain embodiments, the hydrophobic group is selected from methyl, ethyl, propyl, fluoro, and ethylbenzyl.

In certain embodiments, the hydrophobic monomer is an acrylate or acrylamide. In certain embodiments, the hydrophobic monomer is an alkylacrylate. In certain embodiments, the hydrophobic monomer is a haloacrylate. In certain embodiments, the hydrophobic monomer is an alkylalkylacrylate. In certain embodiments, the hydrophobic monomer is a haloaalkylacrylate. In certain embodiments, the hydrophobic monomer is a arylalkylacrylate. In certain embodiments, the hydrophobic monomer is a phenyl acrylate. In certain embodiments, the hydrophobic monomer is a methacrylate. In certain embodiments, the hydrophobic monomer is selected from methylmethacrylate, isobutylmethacrylate, butylmethacrylate, ethylmethacrylate, benzylmethacrylate, cyclohexylmethacrylate, 2-ethylhexylmethacrylate, hexadecylmethacrylate, hexylmethacrylate, octadecymethacrylate, and 2-ethylphenylacrylate. In certain embodiments, the hydrophobic monomer is methylmethacrylate (MMA).

### Swellable monomers

As used herein, the term "swellable monomer" means a monomer that, upon polymerization thereof, has the ability to draw water into the resulting polymer. In certain embodiments, the swellable monomer includes side groups comprising hydrophilic groups. The hydrophilic groups promote polymer swelling by drawing water into the polymer. One of ordinary skill in the art would understand that the degree of polymer swelling will be dependent upon the hydrophilicity of the hydrophilic functional groups on the monomer. One of ordinary skill in the art would understand that the quaternary carbons on the polymer backbone will include two side groups. In certain embodiments, each side group of the quaternary carbons of the swellable monomer comprises a hydrophilic group. In certain embodiments, the swellable monomer comprises quaternary carbons wherein one side group on the quaternary carbons comprises a hydrophilic group and one side group on the quaternary carbons comprises a hydrophobic group. In certain embodiments, the hydrophilic group is selected from the group consisting of hydroxyl, amino, carboxyl, sulfhydryl, carbonyl, amide, pyrrolidone and anhydride. One of ordinary skill in the art would understand that any of the hydrophobic monomers with side groups comprising hydrophobic groups as described above can be modified (or functionalized) with one or more hydrophilic groups to produce a side group comprising a hydrophilic group. By way of non-limiting example, the hydrophobic monomer, ethylmethacrylate can be modified with a hydroxyl group to produce (hydroxyethyl)methacrylate, thus forming a swellable monomer.

In certain embodiments, the swellable monomer is selected from hydroxy(C₂ -C₄ - alkyl)methacrylate, hydroxy(alkyl)acrylate, hydroxy(alkoxyalkyl)methacrylate, hydroxy(alkoxyalkyl)acrylate, alkoxy(alkoxyalkyl)methacrylate, alkoxy(alkoxyalkyl)acrylate, N-(alkyl)acrylamide, N-(alkyl)methacrylamide, N,N-di(alkyl)acrylamide, N,N-(di(alkyl))methacrylamide, vicinal-epoxy(C₁ -C₄ alkyl)methacrylate, vicinal-epoxy(alkyl)acrylate, and alkylacrylate. In certain embodiments, the alkyl group is selected from C₁₋₁₂ alkyl, C₁₋₁₀ alkyl, C₁₋₈ alkyl, C₁₋₆ alkyl, C₁₋₄ alkyl, C₂₋₄ alkyl, C₂₋₆ alkyl, C₂₋₈ alkyl, C₂₋₁₀ alkyl, C₂₋₁₂ alkyl, methyl, ethyl, propyl, butyl, and hexyl, each of which can be linear or branched. In certain embodiments, the alkoxy group is selected from C₁₋₁₂ alkoxy, C₁₋₁₀ alkoxy, C₁₋₈ alkoxy, C₁₋₆ alkoxy, C₁₋₄ alkoxy, C₂₋₄ alkoxy, C₂₋₆ alkoxy, C₂₋₈ alkoxy, C₂₋₁₀ alkoxy, C₂₋₁₂ alkoxy, hydroxymethyl, hydroxyethyl, hydroxypropyl, hydroxybutyl, and hydroxylhexyl, each of which can be linear or branched.

In certain embodiments, the swellable monomer is selected from hydroxy(C₂ -C₄ - alkyl)methacrylate, hydroxy(C₂ -C₄ alkyl)acrylate, hydroxy(C₂ -C₄ alkoxy C₂ - C₄ alkyl)methacrylate, hydroxy(C₂ -C₄ alkoxy C₂ -C₄ alkyl)acrylate, alkoxy(C₂ -C₄ alkoxy C₂ -C₄ alkyl)methacrylate, alkoxy(C₂ -C₄ alkoxy C₂ -C₄ alkyl)acrylate, N-(C₁ - C₄ alkyl)acrylamide, N-(C₁ -C₄ alkyl)methacrylamide, N,N-di(C₁ -C₄ alkyl)acrylamide, N,N-(di(C₁ -C₄ alkyl))methacrylamide, vicinal-epoxy(C₁ -C₄ alkyl)methacrylate, vicinal-epoxy(C₁ -C₄ alkyl)acrylate, and (C₁-C₄alkyl)acrylate.

In certain embodiments, the swellable monomer is selected from 2-(hydroxyethyl)methacrylate (HEMA) (a person of ordinary skill in the art would know that other synonyms for HEMA include, for example, (hydroxyethyl)methacrylate, 2-(hydroxyethyl) methacrylate, 2-hydroxyethyl methacrylate, hydroxyethyl methacrylate and (hydroxyethyl) methacrylate), tetrahydrofurylmethacrylate, N,N-dimethylacrylamide, (2-hydroxypropy)methacrylate (HPMA), methacrylamide, N-methylpyrrolidone and methacrylic acid (MAA). In certain embodiments, the swellable monomer is 2-(hydroxyethyl)methacrylate (HEMA). In certain embodiments, the swellable monomer is tetrahydrofurylmethacrylate. In certain embodiments, the swellable monomer is N,N-dimethylacrylamide. In certain embodiments, the swellable monomer is N-methylpyrrolidone. In certain embodiments, the swellable monomer is methacrylic acid (MAA).

In certain embodiments, the swellable monomer is a non-ionizable swellable monomer. As used herein, a non-ionizable swellable monomer is a swellable monomer that comprises non-ionizable hydrophilic side groups, i.e., hydrophilic side groups that are not capable of being ionized. In certain embodiments, non-ionizable hydrophilic side groups are side groups that are hydrophilic but do not contain an acid moiety. In certain embodiments, non-ionizable swellable monomers are monomers that are hydrophilic but do not contain an acid moiety, i.e., any of the swellable monomers described above that do not contain an acid moiety.

In certain embodiments, the swellable monomer is an ionized or ionizable swellable monomer. As used herein, an ionizable swellable monomer is a swellable monomer that comprises ionizable side groups, i.e., side groups that are capable of being ionized. As used herein, an ionized swellable monomer is a swellable monomer that includes a charged side group at neutral pH. In certain embodiments, the ionizable swellable monomers comprise terminal ionizable groups, including, for example, any ionizable group having a pKₐ of from about 3 to about 8. Examples of ionized or ionizable side groups include, but are not limited to, amines, ammonium salts, carboxylic acids, sulfonic acids, and the like. In certain embodiments, non-ionizable swellable monomers are monomers that are hydrophilic and contain an acid moiety, i.e., any of the swellable monomers described above that contain an acid moiety.

### Polymer Structure and Crosslinkers

One of ordinary skill in the art would recognize that hydrogels are formed from high water content polymers that can include physical or chemical crosslinks (i.e., a bond linking one polymer chain to another) resulting in a network structure. In certain embodiments, the polymers are crosslinked linear polymers. In certain embodiments, the polymers are crosslinked branched polymers. In certain embodiments, the polymers are crosslinked star polymers.

In certain embodiments, the polymers are block copolymers. In certain embodiments, the polymers are alternating copolymers. In certain embodiments, the polymers are periodic copolymers. In certain embodiments, the polymers are random copolymers.

In certain embodiments, the hydrogels are formed from low molecular weight polymers. In certain embodiments, the hydrogels are formed from thermoplastic polymers. In certain embodiments, the hydrogels are formed from polymers. In certain embodiments, the number average molecular weight (Mn) of the polymers that form the hydrogel is from about 2 kDa to about 200 kDa, from about 100 kDa to about 200 kDa, from about 150 kDa to about 200 kDa, from about 75 kDa to about 150 kDa, from about 5 kDa to about 80 kDa, from about 10 kDa to about 75 kDa, from about 20 kDa to about 80 kDa, from about 10 kDa to about 50 kDa, from about 10kDa to about 25 kDa, from about 10 kDa to about 20 kDa, from about 50 kDa to about 100 kDa, from about 2 kDa to about 10 kDa, from about 5 kDa to about 20 kDa, from about 5 kDa to about 15 kDa, or from about 2 kDa to about 10 kDa. In some embodiments, the number average molecular weight (Mn) of the low molecular weight polymers that form the hydrogel is about 2 kDa, about 5kDa, about 8 kDa, or about 10 kDa.

In certain embodiments, the hydrogels are formed from thermoset polymers. In some embodiments, the thermoset polymers have a number average molecular weight of greater than about 200 kDa. A person of ordinary skill in the art would know that thermoset polymers can have an infinite molecular weight.

In certain embodiments, the polymers can have a degree of cross-linking of from about 0 to about 1. In certain embodiments, the polymers can have a degree of cross-linking of about 0.1, about 0.2, about 0.3, about 0.4, about 0.5, about 0.6, about 0.7, about 0.8, about 0.9, or about 0.99. In certain embodiments, the polymers can be fully cross-linked (i.e., a degree of cross-linking of about 1).

In certain embodiments, the hydrogels are formed from chemically crosslinked polymers. In certain embodiments, the time for the hydrogel to swell to equilibrium is a function of crosslinker content. In certain embodiments, an increase in crosslinker will result in an increase time for the hydrogel to swell to equilibrium (due to the decreased ability for water molecules to enter the hydrogel network and form bonds with the hydrophilic groups as well as time to untangle and swell (an entropy issue)).

In some embodiments, the crosslinker is a small molecule. In some embodiments, the crosslinker is a dimer. Suitable crosslinkers include, but are not limited to (tetraethylene glycol)dimethacrylate (TEGDMA), N,N'-methylenebis(acrylamide) (MBA), ethylene glycol diacrylate (EGDA) and PEG diacrylate (PEGDA), tetramethylethylenediamine (TEMED). Additional suitable crosslinkers are dimers of a monomer selected from hydroxy(C₂ -C₄ - alkyl)methacrylate, hydroxy(C₂ -C₄ alkyl)acrylate, hydroxy(C₂ -C₄ alkoxy C₂ - C₄ alkyl)methacrylate, hydroxy(C₂ -C₄ alkoxy C₂ -C₄ alkyl)acrylate, alkoxy(C₂ -C₄ alkoxy C₂ -C₄ alkyl)methacrylate, alkoxy(C₂ -C₄ alkoxy C₂ -C₄ alkyl)acrylate, N-(C₁ - C₄ alkyl)acrylamide, N-(C₁ -C₄ alkyl)methacrylamide, N,N-di(C₁ -C₄ alkyl)acrylamide, N,N-(di(C₁ -C₄ alkyl))methacrylamide, vicinal-epoxy(C₁ -C₄ alkyl)methacrylate, and vicinal-epoxy(C₁ -C₄ alkyl)acrylate. In certain embodiments, the hydrogel is formed from a solution in which the crosslinker is present in weight crosslinker to weight polymer percentage of from about 0.01% to about 1%, from about 0.01% to about 0.9%, from about 0.01% to about 0.8%, from about 0.01% to about 0.7%, from about 0.01% to about 0.6%, from about 0.01% to about 0.5%, from about 0.01% to about 0.4%, from about 0.01% to about 0.3%, from about 0.01% to about 0.2%, or from about 0.01% to about 0.1%. In certain embodiments, the hydrogel is formed from a solution in which the crosslinker is present in an amount of about 0.01%, about 0.02%, about 0.03%, about 0.06%, about 0.09%, about 0.12%, about 0.15%, about 0.18%, about 0.21%, about 0.24%, about 0.27% or about 0.3%.

### Hydrogel Materials

In certain embodiments, the materials disclosed herein are hydrogels that are formed from the polymerization of the above-described monomers. One of ordinary skill in the art would know that as hydrogels come into contact with water, absorption of water molecules into the hydrogel matrix occurs. In certain embodiments, the materials swell as a function of hydration (e.g., as a function of exposure to water in a biological location). In certain embodiments, the degree of swelling will depend on the ratios of the hydrophilic to hydrophobic side groups present in the hydrogel. In certain embodiments, the degree of swelling will depend on the ratios of one or more distinct hydrophilic side groups in the hydrogel. In certain embodiments, the degree of swelling will depend on the ratio of one or more distinct hydrophilic side groups to a plurality of one or more distinct hydrophobic side groups. In certain embodiments, the degree of swelling will depend on the ratio of hydrophobic monomers to swellable monomers in the hydrogel. In certain embodiments, the hydrogels are formed from the co-polymerization of one hydrophobic monomer and one swellable monomer, such as, by way of non-limiting example, polymerization of 2-(hydroxyethyl)methacrylate with methylmethacrylate. In certain embodiments, the hydrogels are formed from the co-polymerization of a non-ionizable swellable monomer and an ionized or ionizable swellable monomer. One of ordinary skill in the art would recognize that the non-ionizable swellable monomer and the ionized or ionizable swellable monomer have side groups that comprise hydrophilic functional groups with varying degrees of hydrophilicity. By way of non-limiting example, the hydrogels can be formed from the co-polymerization of 2-(hydroxyethyl)methacrylate (a non-ionizable swellable monomer) and methacrylic acid (an ionizable swellable monomer). Methacrylic acid comprises an ionizable carboxylic acid group and thus only small amounts of methacrylic acid in the resulting hydrogel will result in large amount of swelling.

In certain embodiments, the hydrogel comprises from about 1% to about 100%, from about 1% to about 90%, from about 1% to about 80%, from about 1% to about 70%, from about 1% to about 60%, from about 1% to about 50%, from about 1% to about 40%, from about 1% to about 30%, from about 1% to about 20%, or from about 1% to about 10% of side groups that comprise hydrophobic groups. In certain embodiments, the hydrogel comprises from about 10% to about 100%, from about 10% to about 90%, from about 10% to about 80%, from about 10% to about 70%, from about 10% to about 60%, from about 10% to about 50%, from about 10% to about 40%, from about 10% to about 30%, from about 10% to about 20%, or from about 5% to about 10% of side groups that comprise hydrophobic groups. In certain embodiments, the hydrogel comprises from about 20% to about 100%, from about 20% to about 90%, from about 20% to about 80%, from about 20% to about 70%, from about 20% to about 60%, from about 20% to about 50%, from about 20% to about 40%, or from about 20% to about 30% of side groups that comprise hydrophobic groups. In certain embodiments, the hydrogel comprises from about 30% to about 100%, from about 30% to about 90%, from about 30% to about 80%, from about 30% to about 70%, from about 30% to about 60%, from about 30% to about 50%, or from about 30% to about 40% of side groups that comprise hydrophobic groups. In certain embodiments, the hydrogel comprises from about 40% to about 100%, from about 40% to about 90%, from about 40% to about 80%, from about 40% to about 70%, from about 40% to about 60%, from about 40% to about 50%, of side groups that comprise hydrophobic groups. In certain embodiments, the hydrogel comprises from about 50% to about 100%, from about 50% to about 90%, from about 50% to about 80%, from about 50% to about 70%, or from about 50% to about 60%, of side groups that comprise hydrophobic groups. In certain embodiments, the hydrogel comprises from about 60% to about 100%, from about 60% to about 90%, from about 60% to about 80%, or from about 60% to about 70% of side groups that comprise hydrophobic groups. In certain embodiments, the hydrogel comprises from about 70% to about 100%, from about 70% to about 90%, or from about 70% to about 80% of side groups that comprise hydrophobic groups. In certain embodiments, the hydrogel comprises from about 80% to about 100%, from about 80% to about 90%, or from about 0% to about 100% of side groups that comprise hydrophobic groups.

In certain embodiments, the hydrogel comprises from about 1% to about 100%, from about 1% to about 90%, from about 1% to about 80%, from about 1% to about 70%, from about 1% to about 60%, from about 1% to about 50%, from about 1% to about 40%, from about 1% to about 30%, from about 1% to about 20%, or from about 1% to about 10% of side groups that comprise hydrophilic groups. In certain embodiments, the hydrogel comprises from about 10% to about 100%, from about 10% to about 90%, from about 10% to about 80%, from about 10% to about 70%, from about 10% to about 60%, from about 10% to about 50%, from about 10% to about 40%, from about 10% to about 30%, from about 10% to about 20%, or from about 5% to about 10% of side groups that comprise hydrophilic groups. In certain embodiments, the hydrogel comprises from about 20% to about 100%, from about 20% to about 90%, from about 20% to about 80%, from about 20% to about 70%, from about 20% to about 60%, from about 20% to about 50%, from about 20% to about 40%, or from about 20% to about 30% of side groups that comprise hydrophilic groups. In certain embodiments, the hydrogel comprises from about 30% to about 100%, from about 30% to about 90%, from about 30% to about 80%, from about 30% to about 70%, from about 30% to about 60%, from about 30% to about 50%, or from about 30% to about 40% of side groups that comprise hydrophilic groups. In certain embodiments, the hydrogel comprises from about 40% to about 100%, from about 40% to about 90%, from about 40% to about 80%, from about 40% to about 70%, from about 40% to about 60%, from about 40% to about 50%, of side groups that comprise hydrophilic groups. In certain embodiments, the hydrogel comprises from about 50% to about 100%, from about 50% to about 90%, from about 50% to about 80%, from about 50% to about 70%, or from about 50% to about 60%, of side groups that comprise hydrophilic groups. In certain embodiments, the hydrogel comprises from about 60% to about 100%, from about 60% to about 90%, from about 60% to about 80%, or from about 60% to about 70% of side groups that comprise hydrophilic groups. In certain embodiments, the hydrogel comprises from about 70% to about 100%, from about 70% to about 90%, or from about 70% to about 80% of side groups that comprise hydrophilic groups. In certain embodiments, the hydrogel comprises from about 80% to about 100%, from about 80% to about 90%, or from about 90% to about 100% of side groups that comprise hydrophilic groups.

In certain embodiments, the hydrogel comprises from about 1% to about 100%, from about 1% to about 90%, from about 1% to about 80%, from about 1% to about 70%, from about 1% to about 60%, from about 1% to about 50%, from about 1% to about 40%, from about 1% to about 30%, from about 1% to about 20%, or from about 1% to about 10% of ionized or ionizable side groups. In certain embodiments, the hydrogel comprises from about 10% to about 100%, from about 10% to about 90%, from about 10% to about 80%, from about 10% to about 70%, from about 10% to about 60%, from about 10% to about 50%, from about 10% to about 40%, from about 10% to about 30%, from about 10% to about 20%, or from about 5% to about 10% of ionized or ionizable side groups. In certain embodiments, the hydrogel comprises from about 20% to about 100%, from about 20% to about 90%, from about 20% to about 80%, from about 20% to about 70%, from about 20% to about 60%, from about 20% to about 50%, from about 20% to about 40%, or from about 20% to about 30% of ionized or ionizable side groups. In certain embodiments, the hydrogel comprises from about 30% to about 100%, from about 30% to about 90%, from about 30% to about 80%, from about 30% to about 70%, from about 30% to about 60%, from about 30% to about 50%, or from about 30% to about 40% of ionized or ionizable side groups. In certain embodiments, the hydrogel comprises from about 40% to about 100%, from about 40% to about 90%, from about 40% to about 80%, from about 40% to about 70%, from about 40% to about 60%, from about 40% to about 50%, of ionized or ionizable side groups. In certain embodiments, the hydrogel comprises from about 50% to about 100%, from about 50% to about 90%, from about 50% to about 80%, from about 50% to about 70%, or from about 50% to about 60%, of ionized or ionizable side groups. In certain embodiments, the hydrogel comprises from about 60% to about 100%, from about 60% to about 90%, from about 60% to about 80%, or from about 60% to about 70% of ionized or ionizable side groups. In certain embodiments, the hydrogel comprises from about 70% to about 100%, from about 70% to about 90%, or from about 70% to about 80% of ionized or ionizable side groups. In certain embodiments, the hydrogel comprises from about 80% to about 100%, from about 80% to about 90%, or from about 90% to about 100% of side groups ionized or ionizable side groups.

In certain embodiments, the hydrogel comprises a polymer with a ratio of hydrophobic monomers to swellable monomers of about 99.09:0.01, 99.9:0.1, 99.5:0.5, 98:2, 95:5, 90: 10, 85:15, 80:20, 75:25, 70:30, 65:35, 60:40, 55:45, or 50:50. In certain embodiments, the hydrogel comprises a polymer with a ratio of swellable monomers to hydrophobic monomers of about 99.09:0.01, 99.9:0.1, 99.5:0.5, 98:2, 95:5, 90:10, 85:15, 80:20, 75:25, 70:30, 65:35, 60:40, 55:45, or 50:50.

In certain embodiments, the hydrogel comprises a polymer formed from a non-ionizable swellable monomer and an ionized or ionizable swellable monomer, wherein the ionized or ionizable swellable monomer has a swellability greater than the non-ionizable swellable monomer. In certain embodiments, the hydrogel comprises a polymer with a ratio of non-ionizable swellable monomers to ionized or ionizable swellable monomers of about 99.09:0.01, 99.9:0.1, 99.5:0.5, 98:2, 95:5, 90:10, 85:15, 80:20, 75:25, 70:30, 65:35, 60:40, 55:45, or 50:50. In certain embodiments, the hydrogel comprises a polymer with a ratio of ionized or ionizable swellable monomers to non-ionizable swellable monomers of about 99.09:0.01, 99.9:0.1, 99.5:0.5, 98:2, 95:5, 90:10, 85:15, 80:20, 75:25, 70:30, 65:35, 60:40, 55:45, or 50:50.

In certain embodiments, the hydrogel comprises a polymer formed from two swellable monomers, wherein the first swellable monomer has a swellability greater than the second swellable monomer. In certain embodiments, the hydrogel comprises a polymer with a ratio of the first swellable monomers to the second swellable monomers of about 99.09:0.01, 99.9:0.1, 99.5:0.5, 98:2, 95:5, 90:10, 85:15, 80:20, 75:25, 70:30, 65:35, 60:40, 55:45, or 50:50. In certain embodiments, the hydrogel comprises a polymer with a ratio of the second swellable monomers to first swellable monomers of about 99.09:0.01, 99.9:0.1, 99.5:0.5, 98:2, 95:5, 90:10, 85:15, 80:20, 75:25, 70:30, 65:35, 60:40, 55:45, or 50:50.

The hydrogel comprises a plurality, e.g., two or more, distinct types of monomers, each comprising side groups with varying degrees of swellablility. The the hydrogel comprises a polymer formed from one or more hydrophobic monomers, and one or more ionized or ionizable monomers. The ratios of the different monomers (and ultimately the relative contribution of the various hydrophilic groups) will depend on the degree of swelling of the material. By way of non-limiting example, a glaucoma shunt may require 100% swelling of the shunt upon hydration, e.g., placement in the eye. One of ordinary skill in the art would understand that depending on the type of device fabricated with the disclosed hydrogels, a different degree of swelling may be desired for the device.

The hydrogels comprise a polymer with the general formula: 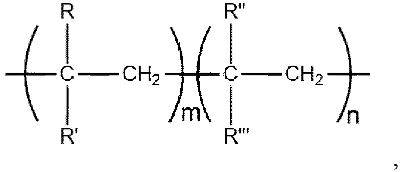 wherein R and R" are methyl, R' is a hydrophobic side group, and R‴ is an ionized or ionizable hydrophilic side group. R' is a non-ionizable hydrophilic side group, and R'" is an ionized or ionizable side group. carboxylic, acid.

R' is -COOR¹ wherein R¹ is an alkyl group.

The ratio of m:n is from about 99.99:0.01 to about 90:10. In certain embodiments, the ratio of m:n is about 99: 1. In certain embodiments, the ratio of m:n is about 98:2. In certain embodiments, the ratio of m:n is about 97:3. In certain embodiments, the ratio of m:n is about 96:4. In certain embodiments, the ratio of m:n is about 95:5. In certain embodiments, the ratio of m:n is about 94:6. In certain embodiments, the ratio of m:n is about 93:7. In certain embodiments, the ratio of m:n is about 92:8. In certain embodiments, the ratio of m:n is about 91:9. In certain embodiments, the ratio of m:n is about 90:10.

### Methods of Making Polymers

The polymer hydrogels can be formed from various methods, including, but not limited to, free radical polymerization and crosslinking. The free radical addition reaction includes an initiator, solvent, crosslinker (as described above), and the monomers (as described above). The initiator can be any free radical initiator, such as thermal initiators, photoinitiators, redox initiators, and the like. Thermal initiators can include, e.g., azo compounds such as 2,2'-azobis(isobutyronitrile) (AIBN) organic peroxides such as benzoyl peroxide (BPO), benzenesulfonic acid esters and alkylsulfonium salts. Certain redox free radical polymerizations can take place under aqueous conditions or in the presence of other solvents such as glycerin, ethylene glycol, aliphatic glycols, formic acid, acetic acid, acetone, and the like. Redox initiators that are used under these conditions include peroxides, persulfates, peroxomonosulfates, peroxidiphosphates, including, e.g., sodium metabisulfite/ammonium peroxydisulfate.

In some embodiments, the hydrogels are synthesized by combining an aqueous solution of an oxygen-purged free radical initiator, a solvent, and a crosslinker with two or more monomers with varying degrees of swellability, then allowing the reaction to polymerize for some time period, e.g., overnight, under ambient pressure and temperature, at an elevated temperature, and/or at an elevated pressure or at a reduced temperature and pressure. In some embodiments, the pressure is elevated to, e.g., 0.69 MPa (100 psi).

In certain embodiments, the polymer hydrogels are formed by a redox free radical addition reaction including aqueous sodium metabisulfite/ammonium peroxydisulfate as a redox free radical initiator, ethylene glycol as a solvent or cosolvent with water, and TEGDMA as a crosslinker.

### Devices

The hydrogels disclosed herein are used for intraocular shunts.

Certain devices, e.g., tubular shunts, can be polymerized in a vessel, such as a tube with a concentric mandrel such that when demolded, the removed mandrel forms a tube. In some embodiments, the tubular device can then be extracted in a solvent, e.g., water, isopropyl alcohol, tetrahydrofuran, and the like.

In certain embodiments, the device can be fabricated by casting in a mold with a mandrel. In certain embodiments, hydrogels formed from thermoset polymers can be used to fabricate the device by casting in a mold with a mandrel. In certain embodiments, hydrogels formed from thermoplastic polymers can be used to fabricate the device by casting in a mold with a mandrel. In certain embodiments, the device can be fabricated by an extrusion process. In certain embodiments, hydrogels formed from thermoplastic polymers with a low degree of cross-linking can be used to fabricate the device by an extrusion process. In certain embodiments, the device can be fabricated with an air mandrel. In certain embodiments, the lumen of the device can be fabricated with a wire whereby the polymer is extruded over a wire to form the lumen. In certain embodiments, hydrogels formed from thermoset cross-linked polymers can be used to fabricate the device by an extrusion process. In certain embodiments, the device can be fabricated using a piston cylinder. In certain embodiments, the device can be fabricated as a rod and a holed drilled concentrically within the rod to form a tube.

In certain embodiments, the device can be dried in the presence of a plasticizer which can be any biocompatible fluid, for example, glycerin, ethylene glycol, and the like. In some embodiments, the biocompatible fluid plasticizer can be added to prevent cracking and to ease rehydration. Accordingly, in some embodiments, the device material further includes a plasticizer. In certain embodiments, the device is contacted with 10% glycerin in 90% water (by weight) such that the glycerin will plasticize the device, and then the device can be dried to remove the water.

The device has a diameter in a dehydrated state and a diameter in a hydrated state, wherein the diameter in the dehydrated state is less than the diameter in the hydrated state. The diameter of the device increases upon hydration of the device from a dehydrated state to a hydrated state. In some embodiments, the diameter increase upon hydration from about 10% to about 500%, from about 20% to about 400%, from about 10% to about 300%, from about 20% to about 300%, from about 25% to about 250%, from about 20% to about 100%, from about 50% to about 150%, from about 50% to about 100%, from about 75% to about 100%, from about 80% to about 100%, from about 90% to about 110%, or from about 100% to about 150%. In some embodiments, the diameter increase upon hydration about 10%, about 20%, about 30%, about 40%, about 50%, about 60%, about 70%, about 80%, about 90%, about 100%, about 110%, about 120%, about 130%, about 140%, about 150%, about 160%, about 170%, about 180%, about 190%, or about 200%. In certain embodiments, the diameter increases upon hydration about 100%.

In certain embodiments, when the device is in the dehydrated state, the device is configured to fit into a needle lumen with a certain diameter. As used herein, the "gauge number" of a needle refers to the Birmingham gauge wire gauge system that specifies the thickness and/or diameter to hypodermic needles and tube products. In certain embodiments, in the dehydrated state, the device is configured to fit into a needle lumen with a gauge number of 20 or greater, 22 or greater, 23 or greater, 24 or greater, 25 or greater, 26 or greater, 27 or greater 28 or greater, 29 or greater, or 30 or greater. In certain embodiments, in the dehydrated state, the device is configured to fit into a 23-gauge needle lumen, In certain embodiments, in the dehydrated state, the device is configured to fit into a 27-gauge needle lumen.

In certain embodiments, the device comprises an inner diameter and an outer diameter. In certain embodiments, the inner diameter of the device is the lumen diameter. In certain embodiments, the outer diameter of the device is the diameter of the outer wall of the device.

In certain embodiments, when the device is in the dehydrated state, the device outer diameter is from about 100 µm to about 500 µm, from about 200 µm to about 400 µm, from about 100 µm to about 300 µm, from about 200 µm to about 350 µm, from about 125 µm to about 175 µm, from about 125 µm to about 150 µm, from about 150 µm to about 175 µm, from about 140 µm to about 160 µm, or from about 145 µm to about 155 µm. In certain embodiments, when the device is in the dehydrated state the device outer diameter is about 150 µm. In certain embodiments, when the device is in the dehydrated state the device outer diameter is about 200 µm.

In certain embodiments, when the device is in the hydrated state the device outer diameter is from about the device outer diameter is from about 120 µm to about 750 µm, from about 240 µm to about 600 µm, from about 120 µm to about 450 µm, from about 240 µm to about 525 µm, from about 150 µm to about 275 µm, from about 125 µm to about 150 µm, from about 150 µm to about 175 µm, from about 140 µm to about 160 µm, from about 500 µm to about 700 µm, from about 400 µm to about 500 µm, from about 300 µm to about 500 µm, from about 300 µm to about 400 µm, or from about 145 µm to about 155 µm. In certain embodiments, when the device is in the hydrated state the device outer diameter is about 180 µm. In certain embodiments, when the device is in the hydrated state the device outer diameter is about 225 µm. In certain embodiments, when the device is in the hydrated state the device outer diameter is about 240 µm. In certain embodiments, when the device is in the hydrated state the device outer diameter is about 300 µm.

In certain embodiments, when the device is in the dehydrated state the device inner diameter is from about 20 µm to about 120 µm, from about 20 µm to about 75 µm, from about 20 µm to about 50 µm, from about 30 µm to about 75 µm, from about 30 µm to about 60 µm, or from about 40 µm to about 50 µm. In certain embodiments, when the device is in the dehydrated state the device inner diameter is about 50 µm.

In certain embodiments, when the device is in the hydrated state the device inner diameter is from about 120 µm to about 250 µm,150 µm to about 250 µm,175 µm to about 250 µm,150 µm to about 200 µm, 175 µm to about 225 µm, or 175 µm to about 200 µm. In certain embodiments, when the device is in the hydrated state the device inner diameter is about 200 µm.

In certain embodiments, the device has a length in a dehydrated state and a length in a hydrated state, wherein the length in the hydrated state is greater than the length in the dehydrated state. In certain embodiments the device length in the dehydrated state is from about 3 mm to about 10 mm, 4 mm to about 10 mm, 5 mm to about 10 mm, 5 mm to about 8 mm, or 4 mm to 9 mm. In some embodiments, the device length in the dehydrated state is about 6 mm.

In certain embodiments the device length in the hydrated state is from about 3.5 mm to about 20 mm, from about 5 mm to about 20 mm, from about 8 mm to about 18 mm, or from about 10 mm to about 20 mm. In certain embodiments, the device length in the hydrated state is about 12 mm.

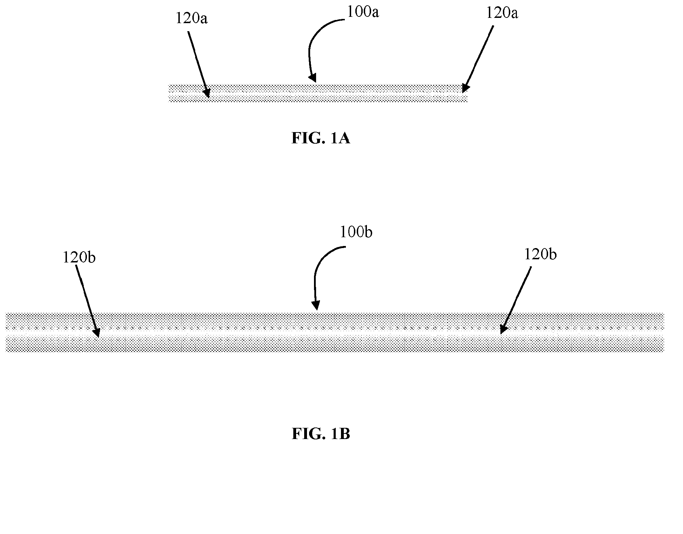

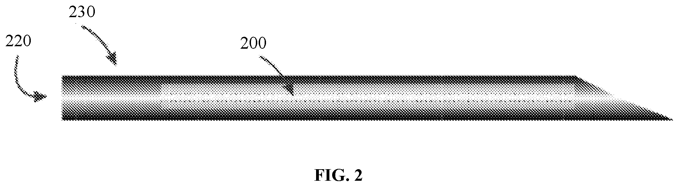

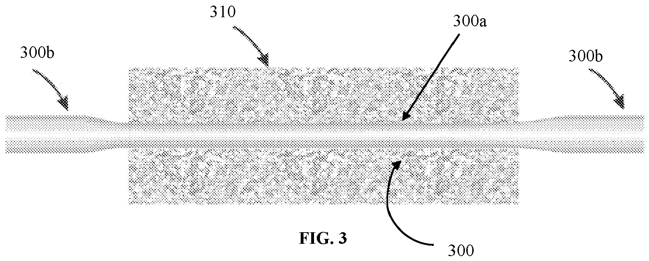

Turning to the figures, **FIG. 1A** shows an intraocular tubular shunt **100a** in a dehydrated state that comprises lumen **120a** extending along the length of tubular shunt **100a.** In certain embodiments, the outer diameter of tubular shunt **100a** is configured to fit into the lumen of a needle of a requisite gauge. **FIG. 1B** shows an intraocular tubular shunt **100b** in a hydrated state in which the lumen **120b**, the length and the outer diameter of the tubular shunt **100b** are greater than the intraocular tubular shunt **100a** in a dehydrated state depicted in **FIG 1A****.** **FIG. 2** shows an intraocular tubular shunt **200** inserted into the lumen **220** of a needle **230.** To insert into the eye of a patient, an inserter (not shown) for the intraocular tubular shunt **200** can be placed in needle **230** and a plunger (not shown) behind the shunt **200** can hold it in place inside the needle **230** as is it inserted through the tissue. **FIG. 3** shows an intraocular tubular shunt **300** placed in tissue **310** in a hydrated state. As shown in **FIG. 3**, the needle (as shown in **FIG. 2**) is used to penetrate tissue **310** and the dehydrated tubular shunt (as shown in, e.g., **FIG. 1**) is deployed across tissue **310.** The plunger that was used to hold the shunt in place in the needle is held steady and the needle is withdrawn, leaving the shunt **300** in place. When hydrated, the ends of the needle **320b** can swell to lock it in place. Similarly, the body of the tube **300a** can swell to seal the needle tract.

### Methods

Provided herein is an intraocular shunt for use in methods of treating glaucoma or ocular hypertension comprising introducing or implanting into a subject in need thereof an intraocular shunt fabricated from the non-degradable swellable hydrogels as disclosed herein..

Provided herein are methods of implanting an ocular device into the eye of a subject. In certain embodiments, the ocular device is a tubular drainage device, e.g., an intraocular tubular shunt. In certain embodiments, the shunt is implanted with one end in the anterior chamber of the eye. In certain embodiments, the shunt is loaded into the lumen of a needle (e.g., between a 22 and 30 gauge needle). In certain embodiments, a plunger is inserted into the lumen proximal to the device. In certain embodiments, the tip of the needle is inserted into the anterior chamber of the eye, and while the plunger is held steady, the needle is withdrawn thereby leaving the shunt in place with its distal end (end furthest away from the user) inside the anterior chamber of the eye. In certain embodiments, the proximal end of the shunt can be placed between the sclera and the conjunctiva or Tenon's capsule of the eye. In certain embodiments, the intraocular shunt can be placed with one end in the anterior chamber and the other in Schlemm's Canal, or the suprachoroidal space or transconjunctival or transcorneal and drain to the outside of the eye. In certain embodiments, the intraocular shunt can be used to shunt aqueous humor from the anterior chamber to any outlet. In certain embodiments, the intraocular shunt can shunt from the anterior chamber to Schlemm's Canal. In certain embodiments, the intraocular shunt can shunt from the anterior chamber to the suprachoroidal space. In certain embodiments, the intraocular shunt can shunt from the anterior chamber to a bleb. In certain embodiments, the intraocular shunt can shunt from the anterior chamber through the conjunctiva. In certain embodiments, the intraocular shunt can shunt from the anterior chamber through the cornea. In certain embodiments, the intraocular shunt can shunt from the anterior chamber through the conjunctiva to the outside of the eye. In certain embodiments, the intraocular shunt can shunt from the anterior chamber through the cornea to the outside of the eye.

In certain embodiments, provided herein is a method of implanting an intraocular shunt into an eye of a subject comprising inserting a tip of a needle into the anterior chamber of the eye of the subject, wherein a lumen of the needle comprises an intraocular shunt, and removing the needle from the eye such that the intraocular shunt remains in the eye. In certain embodiments, the needle comprises a plunger proximal to the intraocular shunt. In certain embodiments, the plunger is removably coupled to the needle, e.g., inside the lumen of the needle. In certain embodiments, the intraocular shunt is removably coupled to the needle, e.g., inside in the lumen of the needle. In certain embodiments, method comprises loading the shunt into the lumen of a needle. In certain embodiments, the shunt to be implanted into the eye is made from a material as disclosed herein.

In certain embodiments, provided herein is disclosed herein is a method of treating glaucoma or ocular hypertension in a subject in need thereof comprising introducing into an eye of the subject an intraocular shunt formed from a material comprising a polymer, the polymer formed from the reaction product of a first monomer comprising a first side group, a second monomer comprising a second side group; and a cross-linking agent, wherein a hydrophilicity of the second side group is greater than a hydrophilicity of the first side group, and wherein the intraocular shunt has a first diameter in a dehydrated state and a second diameter in a hydrated state, wherein the second diameter is greater than the first diameter.

In certain embodiments, provided herein is an intraocular shunt for use in the treatment of glaucoma or ocular hypertension, wherein the intraocular shunt is formed from a material comprising a polymer, the polymer formed from the reaction product of a first monomer comprising a first side group, a second monomer comprising a second side group; and a cross-linking agent, wherein a hydrophilicity of the second side group is greater than a hydrophilicity of the first side group, and wherein the intraocular shunt has a first diameter in a dehydrated state and a second diameter in a hydrated state, wherein the second diameter is greater than the first diameter.

In certain embodiments, provided herein is an intraocular shunt for use in implanting in a subject in need thereof, wherein the intraocular shunt is formed from a material comprising a polymer, the polymer formed from the reaction product of a first monomer comprising a first side group, a second monomer comprising a second side group; and a cross-linking agent, wherein a hydrophilicity of the second side group is greater than a hydrophilicity of the first side group, and wherein the intraocular shunt has a first diameter in a dehydrated state and a second diameter in a hydrated state, wherein the second diameter is greater than the first diameter.

It is to be understood that wherever values and ranges are provided herein, all values and ranges encompassed by these values and ranges, are meant to be encompassed within the scope of the present application. Moreover, all values that fall within these ranges, as well as the upper or lower limits of a range of values, are also contemplated by the present application.

The following examples further illustrate aspects of the present application. However, they are in no way a limitation of the teachings or disclosure of the present application as set forth herein.

### REFERENCE EXAMPLE

### Example 1: Synthesis of poly((2-hydroxyethyl)methacrylate)/methacrylic acid hydrogel.

### Procedure. All percentages are by weight.

Solution A was made of 0.044 M ammonium peroxydisulfate in deionized water (2g/100mL water); Solution B was made of 0.0420 M sodium metabisulfite (4.0 g/100 mL water); Solutions A and B were purged of oxygen by bubbling nitrogen through them for 10 minutes.

The hydrogel was made by combining: 60% purified 2-(hydroxyethyl)methacrylate (HEMA) monomer; %0.2 TEGDMA; variable amount (0%, 1%, 2%, 3%, or 4%) methacrylic acid (MAA); 20% ethylene glycol; 10% Solution A; and 10% Solution B.

All components of the reaction mixture were mixed well. Homogenous gels were formed when the reaction mixture was allowed to polymerize under 0.69 MPa (100 psi) pressure overnight.

The hydrogel was polymerized in a in a 10 mL syringe with a weight placed on the plunger to pressurize the contents of the syringe. The solvent was 20% ethylene glycol and 20% water (from solutions A and B). It was removed from the syringe and hydrated for a few days to rid it of impurities.

### Results.

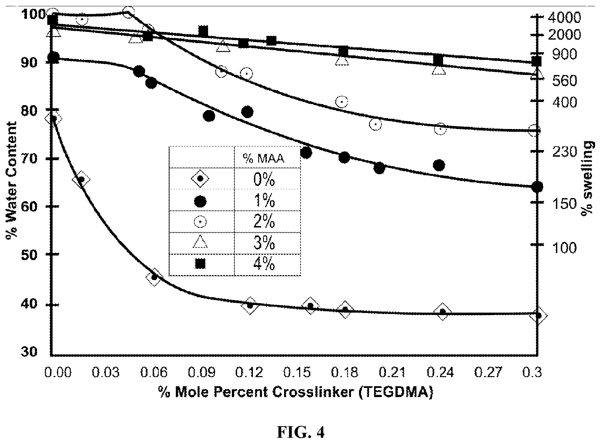

The percent swelling of the hydrogel in saline was a function of the amount of methacrylic acid (MAA) present in the reaction mixture and thus the amount present in the resulting hydrogel. **FIG. 4** shows the results of Example 1. Specifically, **FIG. 4** shows the water content and swollen volume of gels having various mole percent of a ionizable monomer methacrylic acid (MAA) contents versus mole percentage of crosslinker (TEGDMA) in the gel. The base polymer (formed from a non-ionizable monomer) is poly(2-hydroyethyl methacrylate). Percent swelling was determined by weighing the polymer before and after swelling. As 1 mL of water weighs 1 g, the volume change is directly proportional to the weight change.

### Example 2: Swellable pHEMA formulation cast in a pressurized syringe.

Materials. The following materials were used in Example 2: (1) ammonium peroxydisulfalte (Sigma-Aldrich 248614-100g); sodium metabisulfite (Sigma-Aldrich S9000-500g); methacrylic acid (MAA) (Sigma-Aldrich 155721-500g); triethylene glycol dimethacrylate (TEGDMA) (Sigma-Aldrich 759406-10mL); ethylene glycol (EG) (Sigma-Aldrich 324558); and 2-hydroxyethyl methacrylate (HEMA) (Polyscience 04675-100).

Procedure. The ammonium peroxydisulfate solution was prepared by dissolving 2 g of ammonium peroxydisulfate in 100 mL of deionized water and purged of dissolved oxygen by bubbling nitrogen through the solution for five (5) minutes. The sodium metabisulfite solution was prepared by dissolving 4 g of sodium metabisulfite in 100 mL of deionized water and purged of dissolved oxygen by bubbling nitrogen through the solution for five (5) minutes. In a 10 mL polypropylene syringe, 6 mL of HEMA, 0.24 mL of MMA and 0.04 mL of TEGDMA, and 2 mL of EG were mixed and purged of dissolve oxygen by bubbling nitrogen through the mixture for five (5) minutes. 1 mL of the prepared ammonium peroxydisulfate solution and 1 mL of the prepared sodium metabisulfite solution were then added into the 10 mL syringe. The syringe was then shaken to produce a homogeneous solution. The syringe was then kept under pressure by depressing the syringe plunger using a syringe pump at room temperature overnight to allow the solution to polymerize overnight. Then the polymerized pHEMA was removed from the syringe and cut into disks for the swelling test.

Swelling Test. The pHEMA samples from the syringe were incubated in deionized water (DI), phosphate buffered saline (PBS (pH 7.4)), pH 10 solution and pH 4 solution for 48 hours, their swollen weight measured and then vacuum dried to constant weight. The weight changes are reported in the Table 1.

| | DI Water | PBS (pH 7.4) | pH 10 Solution | pH 4 Solution |
|---|---|---|---|---|
| Swollen (g) | 0.975 | 1.03 | 1.585 | 0.845 |
| Vacuum Dry (g) | 0.5 | 0.3 | 0.465 | 0.495 |
| Water Intake (%) | 95.0 | 243.3 | 240.9 | 70.7 |

### Example 3: Preparation of swellable pHEMA tube by casting.

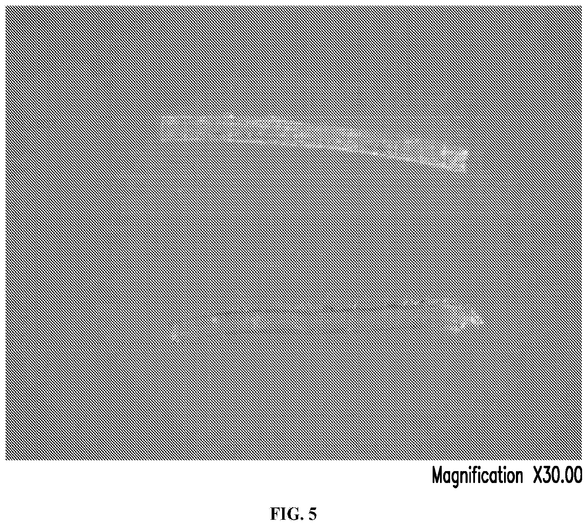

The same formulation from Example 2 was used to prepare a swellable tube by casting in a mold with a mandrel. **FIG. 5** (top) shows the tube after incubation for 1 hour in DI water. **FIG 5** (bottom) shows the tube after incubation for 1 hour in DI water and then vacuum dried to constant weight.

### Example 4: Preparation of swellable pHEMA tube by extrusion.

Procedure. 0.22 g of azobisisobutyronitrile (AIBN), 5.24 g of 2-hydroxyethyl methacrylate (HEMA), and 1.17 g of methacrylic acid (MA) were mixed in a 20 mL vial and allowed to polymerize overnight. The resultant pHEMA was soluble in dimethylformamide (DMF) and compression moldable at 200 °C. A tube was extruded through a plunge extruder.

### Example 5: Fabrication of glaucoma shunt.

Glaucoma shunts are fabricated using the formulations and methods of Examples 1-4 above. Shunts are fabricated with dimensions that allow for use of the shunts in a 27 gauge needle. Shunts are fabricated with dehydrated outer diameters of about 200 µm, dehydrated inner diameters of about 50 µm, and dehydrated lengths of about 6 µm. Shunts are incubated in water resulting in dehydrated outer diameters of about 300 µm, dehydrated inner diameters of about 200 µm, and dehydrated lengths of about 12 µm.

### Example 6: Treatment of glaucoma.

The shunts as fabricated using the formulations and methods of Examples 1-5 are implanted into patients suffering from glaucoma. The shunts are implanted using a 27 gauge needle. The shunts are effective to treat glaucoma. Patients show reduced pressure on the optic nerve, reduced ocular damage, and/or reduced loss of vision, or show a slowing of the progression of glaucoma symptoms as compared to patients who are untreated or who are treated with known devices.

### Example 6: Treatment of ocular hypertension.

The shunts as fabricated using the formulations and methods of Examples 1-5 are implanted into patients suffering from ocular hypertension. The shunts are implanted using a 27 gauge needle. The shunts are effective to treat ocular hypertension. Fluid, e.g., aqueous humor, is redirected and the patients show reduced ocular pressure. Patients show reduced pressure on the optic nerve, reduced ocular damage, and/or reduced loss of vision as compared to patients who are untreated or who are treated with known devices.

## Claims

1. An intraocular shunt formed from a material comprising a non-degrading swellable polymer having the formula: 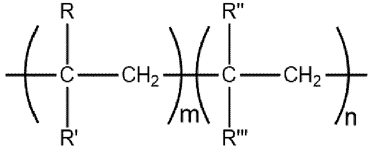 wherein:
R and R" are methyl;
R' is -COOR¹;
R¹ is alkyl; and
R‴ is a carboxylic acid; and
the ratio of m:n is from about 99.99:0.01 to about 90:10;
wherein the intraocular shunt has a first diameter in a dehydrated state and a second diameter in a hydrated state, wherein the second diameter is greater than the first diameter.

2. The intraocular shunt of claim 1, wherein the diameter increases from about 20% to about 200% upon hydration.

3. The intraocular shunt of claim 1, wherein the diameter increases from about 50% to about 100% upon hydration.

4. The intraocular shunt of claim 1, wherein the diameter increases about 100% upon hydration.

5. An intraocular shunt according to any one of claims 1 to 4 for use in a method of treating glaucoma or ocular hypertension in a subject in need thereof comprising introducing or implanting the intraocular shunt into an eye of the subject.

## Patentansprüche

1. Intraokularer Shunt, welcher aus einem Material gebildet ist, umfassend ein nichtabbaubares, quellbares Polymer mit der folgenden Formel: 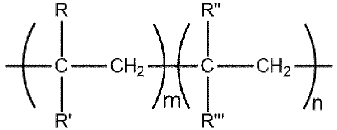 wobei:
R und R" Methyl sind;
R' -COOR¹ ist;
R¹ Alkyl ist; und
R'" eine Carbonsäure ist; und
das Verhältnis von m:n von etwa 99,99:0,01 bis etwa 90:10 beträgt;
wobei der intraokulare Shunt einen ersten Durchmesser in einem dehydrierten Zustand und einen zweiten Durchmesser in einem hydratisierten Zustand aufweist, wobei der zweite Durchmesser größer als der erste Durchmesser ist.

2. Intraokularer Shunt gemäß Anspruch 1, wobei der Durchmesser bei Hydratation um etwa 20 % bis etwa 200 % zunimmt.

3. Intraokularer Shunt gemäß Anspruch 1, wobei der Durchmesser bei Hydratation um etwa 50 % bis etwa 100 % zunimmt.

4. Intraokularer Shunt gemäß Anspruch 1, wobei der Durchmesser bei Hydratation um etwa 100 % zunimmt.

5. Intraokularer Shunt gemäß einem der Ansprüche 1 bis 4 zur Verwendung in einem Verfahren zur Behandlung eines Glaukoms oder einer okulären Hypertension bei einem Patienten, welcher diese benötigt, umfassend das Einführen oder Implantieren des intraokularen Shunts in ein Auge des Patienten.

## Revendications

1. Une dérivation intraoculaire formée à partir d'un matériau comprenant un polymère gonflable non dégradable ayant la formule: 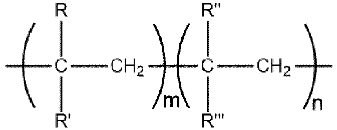 dans laquelle:
R et R" sont des méthyles;
R' est -COOR¹;
R¹ est un alkyle; et
R'" est un acide carboxylique; et
le rapport m:n est compris entre environ 99,99:0,01 et environ 90:10;
dans laquelle la dérivation intraoculaire a un premier diamètre à l'état déshydraté et un second diamètre à l'état hydraté, dans laquelle le second diamètre est plus grand que le premier diamètre.

2. La dérivation intraoculaire selon la revendication 1, dans laquelle le diamètre augmente d'environ 20 % à environ 200 % en cas d'hydratation.

3. La dérivation intraoculaire selon la revendication 1, dans laquelle le diamètre augmente d'environ 50 % à environ 100 % en cas d'hydratation.

4. La dérivation intraoculaire selon la revendication 1, dans laquelle le diamètre augmente d'environ 100 % en cas d'hydratation.

5. Une dérivation intraoculaire selon l'une quelconque des revendications 1 à 4 pour l'utilisation dans un procédé de traitement du glaucome ou de l'hypertension oculaire chez un sujet qui en a besoin, comprenant l'introduction ou l'implantation de la dérivation intraoculaire dans un oeil du sujet.
